# EUROPÄISCHE PATENTSCHRIFT

(11) **EP 3 212 127 B1**
(45) Veröffentlichungstag und Bekanntmachung des Hinweises auf die Patenterteilung: **17.04.2019**
(21) Anmeldenummer: 15808329.5
(22) Anmeldetag: 15.10.2015
(51) Int. Cl.: A61F 2/30, A61F 2/78, A61F 2/28, A61F 2/80, A61F 2/76

(54) **PROTHESENSYSTEM**
PROSTHETIC SYSTEM
SYSTÈME DE PROTHÈSE

(30) Priorität: 28.10.2014 DE 102014115676
(43) Veröffentlichungstag der Anmeldung: 06.09.2017
(73) Patentinhaber: Zarling, Sven, 37115 Duderstadt (DE); Föllmer, Dieter, 17493 Greifswald Hansestadt (DE)
(72) Erfinder: Zarling, Sven, 37115 Duderstadt (DE); Föllmer, Dieter, 17493 Greifswald Hansestadt (DE)
(74) Vertreter: Murgitroyd & Company
(86) Internationale Anmeldenummer: PCT/DE2015/100428
(87) Internationale Veröffentlichungsnummer: WO 2016/066158

(56) Entgegenhaltungen:
- WO-A1-03/065938
- WO-A1-2007/001223
- GB-A- 2 468 967
- US-B1- 7 922 773

## Beschreibung

Die Erfindung betrifft ein Prothesensystem für menschliche Gliedmaßen aufweisend ein Implantat mit mindestens zwei zueinander bewegbaren Gelenkteilen, wobei beide Gelenkteile ausgelegt sind, intrakorporal angeordnet zu werden. Die Erfindung betrifft zudem ein Prothesensystem umfassend ein subkutanes Implantat und einen Schaft.

### Technologischer Hintergrund

Prothesensysteme für menschliche Gliedmaße nach Amputation oder Exartikulation sind seit langem bekannt. Diese Prothesen können als intrakorporale Implantate verwendet sein, die mit einem Knochenteil verbindbar und von Körpergewebe umgeben sind. Diese Endoprothesen oder Implantate sind für einen langfristigen Gebrauch eines Patienten geeignet und können nach Ausheilung des Gliedmaßenstumpfes mit außerhalb des menschlichen Körpers angebrachten Gliederprothesen sogenannten Exoprothesen verbunden werden. Beispiele für künstliche Glieder nach einer Amputation am Oberschenkel sind Kniegelenk-, Unterschenkel- und Fußprothesen. Bei Amputationen oder Exartikulationen im Bereich der oberen Extremität werden Oberarm- und/oder Unterarmprothesen benötigt. Aus dem Patent GB 2 48 967 A ist ein perkutanes Implantat bekannt, das durch die Haut hindurchgeführt wird, um mit einer Exoprothese verbunden zu werden, und als Alternative zu einer Schaftprothese dienen kann.

Üblicherweise erhalten die Patienten sogenannte Schaftprothesen, wobei der Schaft den Stumpf allseitig umgibt und dazu dient, die Kraft des Stumpfes auf wenigstens ein künstliches Glied einer Exoprothese zu übertragen. Ein kritischer Punkt für die Funktion einer Prothese ist die Kraftübertragung. In einem Schaft für Beinprothesen wirken verschiedene Kräfte wie Stoß-, Rotations- und Scherkräfte. Insbesondere beim Laufen besteht das Problem, dass bei der Kraftübertragung vom Schaft auf den Weichteilmantel Kräfte auftreten, die zu Schwellungen, Druckstellen und Entzündungen am Beinstumpf führen können.

### Beschreibung der Erfindung

Auf Grundlage der vorliegenden Erfindung sollen die oben genannten Nachteile von bekannten Schaftversorgungen reduziert und eine schnellere Versorgung von amputierten Patienten durch schnelleres Auffinden eines passgenauen Schaftes gewährleistet werden. Dabei soll die Kraftübertragung von einem Schaft in das Prothesensystem optimiert werden.

Weiterhin soll die Ausbildung der verbliebenen Muskulatur im Stumpf nicht wie bei herkömmlichen Prothesensystemen eingeschränkt werden. Ferner ist es eine Aufgabe der vorliegenden Erfindung eine Nutzung der Muskeln für eine Steuerung von Prothesenkomponenten zu ermöglichen.

Mit der vorliegenden Erfindung wird außerdem das Ziel verfolgt, den Tragekomfort eines Schaft- Prothesensystems zu verbessern.

Vor diesem Hintergrund ist es die Aufgabe der vorliegenden Erfindung, ein verbessertes Prothesensystem bereitzustellen.

Gelöst wird die Aufgabe durch ein Prothesensystem umfassend ein subkutan anzuordnendes Implantat mit den Merkmalen des Anspruches 1. Vorteilhafte Weiterbildungen ergeben sich aus den Unteransprüchen.

Erfindungsgemäß wird ein Prothesensystem für menschliche Gliedmaße bereitgestellt, das ein Implantat mit mindestens zwei zueinander bewegbaren Gelenkteilen aufweist, wobei das erste Gelenkteil mit einem Knochenteil verbindbar ist und das zweite Gelenkteil auf dessen gelenkfernen Seite ein subkutan anzuordnendes Endteil aufweist.

Durch die Bereitstellung eines gelenkigen Endteiles des Implantates kann das intrakorporale Endteil Lasten bzw. Kräfte aus dem Stumpf besser aufnehmen. Zu kontrollierende Kräfte entstehen insbesondere bei Nutzung einer angeschlossenen extrakorporalen Prothese. Die beiden Gelenkteile bilden eine bewegliche Verbindung, wobei geeignete einachsige, zweichachsige oder dreichachsige Gelenkformen wie z.B. Filmscharniere, Ellipsoidgelenke oder Kugelgelenke verwendet werden können.

Wird das Implantat beispielsweise mit einem Oberschenkelknochen verbunden, kann vorteilhafterweise die natürliche Neigung des Oberschenkelknochens durch die Beweglichkeit des zweiten Gelenkteiles ausgeglichen werden.

Auch Kräfte, die bei Armamputierten am Stumpfende einwirken, können durch ein gelenkiges Endteil des Implantates besser aufgenommen werden. Beispielsweise können axiale sowie nicht axiale Kräfte, die durch das Gewicht einer Unterarmprothese oder bei Liegestützen auftreten, besser in den Armstumpf übertragen werden.

Gemäß einer vorteilhaften Weiterbildung des Prothesensystem ist das Endteil des zweiten Gelenkteiles plattenförmig ausgebildet.

Das plattenförmige Endteil oder die Endplatte kann integral mit dem zweiten Gelenkteil verbunden sein oder mit geeigneten Besfestigungsmitteln an dem zweiten Gelenkteil befestigt werden. Die Endplatte kann beispielsweise angeschraubt werden. Dabei kann die Form der Endplatte so gewählt werden, dass die Endplatte der Größe des Stumpfendes angepasst werden kann.

Auf diese Weise kann die Belastungsfläche vergrößert werden und auftretende Kräfte können sich vorteilhaft auf der Fläche verteilen. Dies steht im Gegensatz zu einer relativ kleinen Schnittfläche eines Knochenstumpfes, der Kräfte lediglich punktuell aufnehmen kann. Derartige Punktlasten verursachen Schmerzen und gegebenfalls Gewebeschädigungen. Daher wurde konventionell mit einem erhöhten konstruktiven Aufwand, das Körpergewicht am Tuber abgefangen.

Mittels der bewegbar gelagerten Endplatte können aufwendige Konstruktionen zur Verlagerung der Lastaufnahmefläche in den Tuberbereich sowie Punktlasten und damit verbundene Schäden der Weichteilmasse vermieden werden. Die Belastbarkeit des Amputationsstumpfes wird durch die Bereitstellung eines vorzugsweise kantenfreien Endteils des zweiten Gelenkteiles signifikant verbessert, da Druckstellen und Verletzungen der Weichteile reduziert werden können.

Durch das definierte Endteil des zweiten Gelenkteiles ist die Lage des distalen Endes des Knochens einfach auffindbar. Bei herkömmlichen Prothesen ist dagegen die Lage des Knochenschaftendes im Weichteilbereich nicht immer problemlos lokalisierbar und kann gegebenenfalls die Gefahr beinhalten, dass Weichteilgewebe durchbohrt wird.

Bei einer vorbestimmten Endplattenform ist vorteilhafterweise eine individuelle Abformung der Gliedmaßenstümpfe nicht mehr notwendig, sodass eine schnelle Patientenversorgung mit einem zum Endteil passenden Gegenstück in einem Schaft erfolgen kann. Der Abschluss des Stumpfes mit einer vorbestimmten Außenform des beweglichen Endteiles, ermöglicht es, zeitbedingte mögliche Veränderungen des Weichteilgewebes im distalen Stumpfbereich zu vernachlässigen. Dadurch kann vorteilhafterweise auf aufwendige Nacharbeiten oder Neuanpassungen des Schaftes nach gewissen Tragezeiten verzichtet werden.

Eine Weiterbildung der Erfindung sieht vor, dass auf die Außenseite des zweiten Gelenkteiles eine anatomisch angepasste Kontaktfläche gebildet ist. Mit Hilfe der anatomisch angepassten Kontaktfläche wird ermöglicht, dass die Haut auf eine möglichst natürliche Weise über die Fläche gleiten kann. Die Fläche unterstützt eine passgenaue Integration der Prothese mit einem Gegenstück des Schaftes. Mit Hilfe der anatomisch angepassten Kontaktfläche ohne scharfe Kanten werden Verletzungen und Druckstellen vermieden und im Zusammenspiel mit einem entsprechend ausgebildeten Gegenstück eines Schaftes kann ein Verrutschen der intrakorporalen Prothese zum Schaft bzw. der daran anschließbaren extrakorporalen Prothese vermieden werden.

In einer bevorzugten Weiterbildung des Prothesensystems weist das Endteil eine konvex gekrümmte Außenfläche auf.

Auf diese Weise kann eine gleichmäßige Kraftübertragung zwischen dem Endteil und einem vorzugsweise konkaven Gegenstück in einem Schaft erfolgen, wodurch Druckstellen am Körper vermieden werden.

Die konvex gekrümmte Außenfläche hat den weiteren Vorteil, dass die Haut auf ihr gleitbeweglich anliegen kann. In anderen Worten kann nach einer subkutanen Anordnung des Implantates die Haut leicht über die bevorzugt glatt ausgebildete Außenfläche des Endteiles gleiten.

In einer bevorzugten Weiterbildung des Prothesensystems ist das erste Gelenkteil über einen Marknagel und/oder einer Verschraubungseinheit mit einem Knochenteil verbindbar.

Mit einem Marknagel und/oder einer Verschraubungseinheit kann das erste Gelenkteil in den Knochenkanal eines abgeschnittenen Knochenschaftes eingeführt und somit verbunden werden. Die Verbindung oder Verankerung erfolgt entweder mittels Knochenzement oder andere geeignete Befestigungsmittel. Weiterhin ist auch eine Verankerung ohne Befestigungsmittel denkbar.

Mittels eines Marknagels kann der Gliedmaßenstumpf auf die optimale Länge gebracht werden. Auf diese Weise kann beispielsweise eine Beinlängendifferenz oder Oberschenkellängendifferenz im Vergleich zur normalen Bein- bzw. Oberschenkellänge ausgeglichen werden. Mit Hilfe eines Marknagels kann die Länge des Stumpfes variiert werden und an die Weichgewebesituation angepasst werden. Besteht ein Weichteilüberhang nach einer Amputation kann der Marknagel diesen überbrücken.

In einer bevorzugten Weiterbildung des Prothesensystems ist das erste Gelenkteil in Form eines Gelenkkopfes und das zweite Gelenkteil in Form einer Gelenkpfanne ausgebildet.

Die Verwendung eines Kugelgelenkes hat den Vorteil einer Bewegungsfreiheit von drei Freiheitsgraden. So ist ein dreiachsiges Kugelgelenk beweglicher als ein einachsiges Scharnier. Auf diese Weise kann das bewegliche Gelenkteil auf Krafteinwirkungen aus verschiedenen Richtungen optimal reagieren.

Das Kugelgelenk kann eine konkave kleinere Gelenkfläche aufweisen, die Kontakt mit einer konvexen größeren Gelenkfläche hat. Die Gelenkpfanne kann dabei mit einem ringförmigen Überstand mit Federmitteln ausgebildet sein, sodass die Gelenkkugel nicht ohne Kraftaufwand aus der Gelenkpfanne springen kann. Auf diese Weise kann das erste und zweite Gelenkteil eine gekoppelte Einheit bilden, deren Gelenkteile unabhängig von Muskelanbindungen oder Lage zusammengehalten werden können. Für eine derartige Fixierung der Gelenkteile zueinander können bekannte Steck- oder Schnappmechanismen oder dergleichen verwendet werden, wobei diese Fixierungsmechanismen das Gleiten der Gelenkteile zueinander möglichst nicht einschränken sollten.

In einer bevorzugten Weiterbildung des Prothesensystems ist das erste Gelenkteil hohlförmig ausgebildet und weist Befestigungsmittel auf, die ausgewählt sind aus der Gruppe umfassend knochenaufbauendes Material, Knochenzement und Haftmittel.

Handelt es sich beim ersten Gelenteil um einen kugelförmigen Gelenkkopf, kann die Kugelschale des Gelenkkopfes über das distale Schaftende gestülpt werden. Dabei ermöglichen geeignete Befestigungsmittel in der Kugelschale ein Verwachsen oder Verkleben des ersten Gelenkteiles mit dem Knochenschaft.

In einer bevorzugten Weiterbildung des Prothesensystems weist das Endteil einen radial umlaufenden Randbereich auf.

Mittels des radial umlaufenden Randbereiches wird die gebildete Auflagefläche vergrößert. Dabei kann der Außenumfang der bevorzugt halbkugelförmigen Endplatte wenigstens das Anderthalbfache des Außendurchmessers des distalen Endes des Knochenschaftes betragen. Die vergrößerte Auflagefläche verringert die Druckbelastung durch einen den Gliedmaßenstumpf umhüllenden Schaft auf noch vorhandene Sehnen und Muskeln. Eine dauerhafte Komprimierung der Weichteile und insbesondere der Muskulatur kann durch das Bereitstellen eines Randbereiches verhindert werden. Die Muskulatur kann sich optimalerweise so ausbilden, wie sie es bei den entsprechenden gesunden Gliedmaßen tun würde.

Vorteilhafterweise können im radialen Randbereich, Muskeln und/oder Sehnen angebunden werden. Damit kann die Wirkungsstärke der vorhandenen Restmuskulatur zumindest teilweise aufrecht erhalten werden. Die Muskeln werden durch die Anordnung einer Endplatte mit einem radialen Randbereich nicht unnatürlich umgelenkt, wie es bei einer Prothese ohne überstehende Endplatte der Fall wäre.

Mit Hilfe des radialen Randbereiches ist die Außenfläche des Endteiles größer als der Außenumfang des ersten Gelenkteiles, so dass Kräfte besser eingeleitet und verteilt werden können.

In einer bevorzugten Weiterbildung des Prothesensystems weist das Endteil des zweiten Gelenkteiles Flächen und/oder Material zur Anbindung von Muskeln auf.

Durch eine feste Anbindung von Muskeln, die für die Extension bzw. Beugung der amputierten Gliedmaßen verwendet werden können, kann wenigstens ein Teil der Restmuskulatur funktionell genutzt werden. Die Muskeln können an geeignet ausgebildeten Flächen wie z.B. feinporigen Verankerungsflächen anwachsen. Alternativ oder zusätzlich kann das Einwachsen von Muskelgewebe erleichtert werden, indem das zweite Gelenkteil mit einem Gewebe oder mit Gittern aus Metall oder anderen geeigneten Materialien belegt wird. Nach erfolgreichem Anwachsen oder Verwachsen des Muskelgewebes kann die muskuläre Funktion und Stabilität der angebundenen Muskeln wiederhergestellt werden. Die Anbindung der vorhandenen Muskulatur ermöglicht eine Bewegung des gelenkigen Endteiles bzw. der Endplatte. Durch das willentlich durch Muskeln steuerbare zweite Gelenkteil kann eine weitere extrakorporale Prothese wie eine Knie- oder Fußprothese gesteuert werden.

Durch Aufrechterhalten des Muskelvolumens kann ein Schwimmen des Knochenstumpf im Weichteilmantel bzw. des gesamten Stumpfes im Prothesenschaft verhindert werden. Dies gewährleistet eine höhere Sicherheit und besseren Tragekomfort für den Nutzer des Prothesensystems.

In einer bevorzugten Weiterbildung des Prothesensystems umfasst das Prothesensystem einen den Gliedmaßenstumpf wenigstens teilweise umhüllenden Schaft zur Anbindung an extrakorporale Prothesenmechanismen.

Vorteilhafterweise weist der Schaft eine Gegenplatte auf, dessen Form an die Außenform des zweiten Gelenkteiles angepasst ist.

Die vorbestimmte Form der Endplatte im Zusammenspiel mit der passenden Gegenplatte im Schaft hat den Vorteil, dass eine individuelle Formanpassung des Schaftes nicht mehr notwendig ist. Auf diese Weise können passgenaue, industriell hergestellte Schaftgegenstücke verwendet werden und die Versorgung des Patienten erleichtern. Kurze Zeit nach Abheilung des Stumpfes kann der Schaft angepasst werden. Die Anpassung kann optimaler Weise in einem Zeitraum von wenigen Stunden erfolgen. Diese Zeitersparnis kann die Kosten im Vergleich zu Prothesen senken, die langwierig und wiederholt angepasst werden müssen. Ein weiterer Vorteil des Prothesensystems ist, dass der gesamte Versorgungsverlauf, einen Krankenhaus- und Rehabilitationsaufenthalt eingeschlossen, zeitlich reduziert werden kann.

Aufgrund einer Formanpassung der Gegenplatte im Schaft an die Außenform der subkutanen Endplatte kann hier die Hauptkraftübertragung erfolgen, sodass der obere Schaftrand im Tuberbereich nicht mehr oder nur teilweise als Abstützung benötigt wird. Daher kann der Schaft in einer bevorzugten Weiterbildung des Prothesensystems als teilweise offener Rahmenschaft ausgebildet werden.

Die offene Konstruktion des Schaftes anstelle einer geschlossenen Containerkonstruktion ermöglicht, einen besseren Luft- und Temperaturaustausch mit der Umgebung. Damit wird in der Regel der Schweißbildung vorbeugt. Dies verbessert für den Nutzer den Tragekomfort. Zudem können Druckstellen vermieden werden.

In einer bevorzugten Weiterbildung des Prothesensystems kann der Schaft ein zirkulär verlaufendes Band als Haltevorrichtung für den Schaft aufweisen.

Das zirkuläre Band bildet eine Bandage, dessen Umfang variabel je nach Stumpfumfang verstellbar ist. Dies ermöglicht einen guten Halt des Schaftes. Der Schaft ist durch das verstellbare Band als Haltervorrichtung für einen Schaft auch für die Anwendung im Wasser geeignet.

Zur Befestigung des Bandes an den Schaft weist die Außenseite des Bandes Haltemittel auf, die mit entsprechenden Haltemitteln des Schaftes formschlüssig zusammenwirken können.

Ferner kann das Band Messsensoren für Myosignale aufweisen. Myosignalsensoren können im Band auf der der Haut zugewandten Seite oder alternativ auf der freien Hautoberfläche angeordnet sein, um die Anspannung der Beuge- bzw. Streckmuskeln zu erfassen. Messbar sind myoelekrische Signale beispielsweise über Elektroden auf der Haut. Dabei sind myoelektrische Signale elektrisch messbare Signale, die bei der bewussten und unbewussten Kontraktion von Muskeln entstehen. Die Messung der Muskelbewegungen kann vorteilhafterweise für die Steuerung des Prothesensystems genutzt werden, um Bewegungen der Exoprothese wie z. B. Kniebeugen oder bei der Hand Greifbewegungen zu steuern.

In einer bevorzugten Weiterbildung des Prothesensystems wird wenigstens ein externes Messsystem bereitgestellt, das Messsensoren zum Erfassen der Position des zweiten Gelenkteiles aufweist. Dabei kann das wenigstens eine externe Messsystem vorzugsweise unterhalb des Stumpfes als Gegenplatte und/oder als Rohrelement ausgebildet sein.

Auf diese Weise kann auf Messsensoren auf der Haut oder im Band verzichtet werden. Es reicht z. B. aus, mittels der Messsensoren in der Gegenplatte die Lage des zweiten Gelenkteiles zu erfassen. Damit kann indirekt die Bewegung der Endplatte durch Muskeln gemessen werden. Werden elektrische Kabelverbindungen zum Abnehmen und Weiterleiten der erfassten Messsignale verwendet, können diese in der Schaftkonstruktion integriert werden.

Alternativ oder zusätzlich zu einem externen Messsystem kann wenigstens ein internes Messsystem bereitgestellt werden. Für interne Messungen im Implantat weist beispielsweise das zweite Gelenkteil Messsensoren auf, um die Position des zweiten Gelenkteiles zu erfassen. Diese Messsensoren können die erfassten Daten kabellos an eine Steuereinheit senden. Ein in der Endplatte integrierte Sensorik kann die Funktionalität der extrakoporalen Prothese gewährleisten. Die Messsensoren im Implantat können auch zusätzlich zu einem oder mehreren Sensoren in der Gegenplatte vorhanden sein, um Signale für die Steuerung und/oder Belastungsmessungen der intrakorporalen Komponenten nutzen zu können.

In einer bevorzugten Weiterbildung des Prothesensystems weist das Prothesensystem eine Steuereinheit auf, wobei auf Grundlage der erfassten Messsignale extrakorporale Prothesenmechanismen steuerbar oder regelbar sind.

Auf Grundlage der durch diese Messsensoren erfassten Positionsdaten der Gelenkplatte (z. B. Status nach vorne gekippt oder nach hinten gekippt) kann eine angeschlossene Exoprothese gesteuert werden.

Die genannten Kippbewegungen können nach dem Einbau der Prothese und Anbindung der Muskeln an das Endteil durch den Nutzer selbst gesteuert werden. Durch die direkte oder indirekte Messung der Lage des subkutan angeordneten zweiten Gelenkteiles können auf einfache Weise die Bewegungsabsichten des Nutzers an die extrakorporale Prothese weitergeleitet werden.

Die Erfindung stellt nicht nur ein optimal sitzendes, gut belastbares Prothesensystem bereit, sondern gleichzeitig auch eine Möglichkeit die extrakorporale Prothese gut steuern zu können. Dabei ist es Ziel, die natürlichen Muskeln, wenn vorhanden, möglichst weiter zu nutzen, oder über einen externen Trigger nutzbar zu machen. Durch eine bessere Funktionalität des Prothesensystems, wird die Beweglichkeit und Selbständigkeit des Betroffenen erhöht.

### Kurze Beschreibung der Figuren

Weitere Vorteile und Merkmale der Erfindung ergeben sich aus der nachfolgenden Beschreibung von Ausführungsbeispielen anhand der Figuren. Es zeigen:
Figur 1 eine Schnittansicht eines Prothesensystems für einen Oberschenkel;
Figur 2 eine weitere Ausführungsform eines Femurprothesensystems umfassend einen Marknagel;
Figur 3 eine weitere Ausführungsform eines Femurprothesensystems umfassend einen Marknagel;
Figur 4 eine weitere Ausführungsform des Prothesensystems für einen Unterschenkel;
Figur 5 eine weitere Ausführungsform eines Femurprothesensystems;
Figur 6 eine weitere Ausführungsform eines Femurprothesensystems umfassend einen Schaft;
Figur 7 Schnittansicht einer weiteren Ausführungsform eines Femurprothesensystems umfassend einen Schaft mit einem externen Messsystem mit Sensoren zur Erfassung der Lage und Belastung des subkutanen Gelenkteiles des Implantates;
Figur 8 Schnittansicht einer weiteren Ausführungsform eines Prothesensystems mit einem weiteren externen Messsystem, das unter dem Schaft angebracht ist; und Figur 9 eine schematische Draufsicht eines externen Messsystems in Form einer Gegenplatte .

### Detaillierte Beschreibung der Figuren

Die Darstellungen sind schematisch und nicht zwingend maßstabsgerecht. Sie zeigen darüber hinaus nicht alle Details, sondern beschränken sich auf die Darstellung der erfindungswesentlichen Einzelheiten sowie weiterer Merkmale, die die Erläuterung und Beschreibung der Erfindung erleichtern. Gleiche Elemente in den unterschiedlichen Figuren werden mit den gleichen Bezugszeichen bezeichnet.

Im Folgenden werden Ausführungsformen der Erfindung anhand der Zeichnungen näher erläutert.

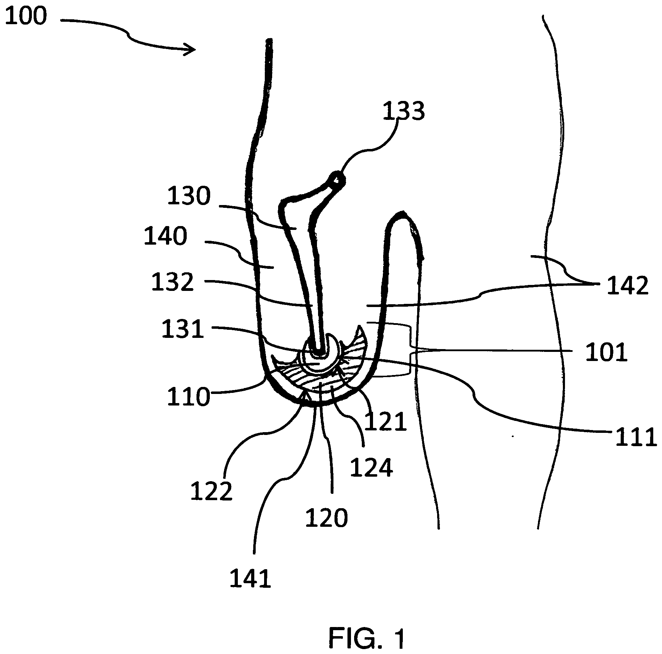

Figur 1 zeigt ein Prothesensystem 100 in Form einer Oberschenkelprothese. Der Oberschenkel 142 besitzt einen abgetrennten Femurknochen 130. Die Schnittfläche des Knochenschaftes 132 befindet sich am distalen Schaftende 131. Der Femurkopf 133 lagert in der Hüftpfanne, die nicht gezeigt ist. Der Femurknochen 130 wird von einem Weichteilmantel 140 umgeben.

Das Implantat 101 ist ausschließlich intrakorporal angeordnet, wobei das erste Gelenkteil 110 mit dem distalen Schaftende 131 mit Knochenzement oder zementfrei verbunden ist. Das erste Gelenkteil 110 ist als Gelenkkopf mit einer konvexen Gleitfläche 111 ausgebildet. Die Gleitfläche 111 des Gelenkkopfes steht mit der Gleitfläche 121 des zweiten Gelenkteiles 120 in Kontakt. Das zweite Gelenkteil 120 kann um den Gelenkkopf des ersten Gelenkteiles 110 bewegt werden, sodass das zweite Gelenkteil 120 verschiedene Lagen annehmen kann.

Auf der gelenkfernen Seite weist das zweite Gelenkteil eine Außenfläche 122 auf, die subkutan angeordnet ist. Die Außenfläche 122 ist konvex gekrümmt und kann Druck bzw. Gewichtskräfte aufnehmen. Durch die Krümmung kann die Haut leicht über das Endteil gleiten.

Das zweite Gelenkteil 120 weist ein integrales Endteil 124 auf. Die Implantatkomponenten können aus verschiedenen geeigneten Implantatsmaterialien ausgebildet sein. Dabei können die Materialien starr und/oder flexibel sein. Eine flexible Ausführung des radialen Randbereiches des Endteiles 124 kann in diesem Bereich auftretende Stöße dämpfen. Weiterhin kann das gesamte Endteil 124 als Stoßdämpfer ausgebildet sein, um am distalen Amputationsstumpfende 141 auftretende Stöße dämpfen zu können.

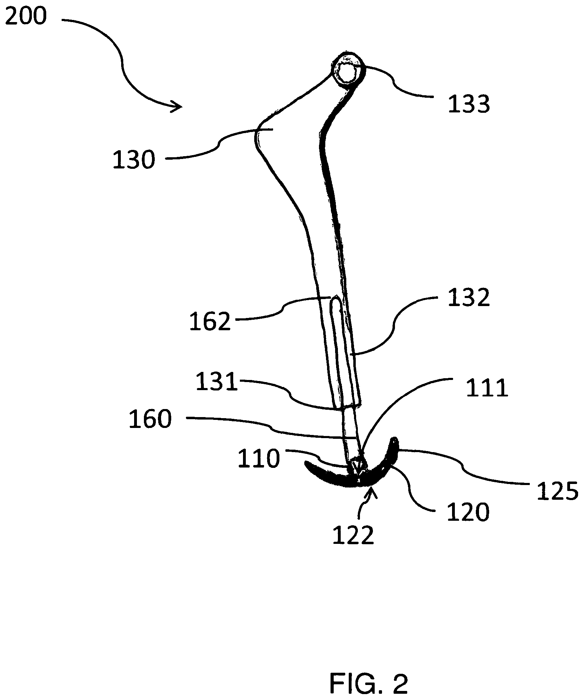

Figur 2 zeigt eine weitere Ausführungsform eines Femurprothesensystems 200, wobei das erste Gelenkteil 110 über einen Marknagel 160 mit dem Femurknochen 130 verankert wird. Dabei wird der Marknagel 160 in den Knochenkanal des abgeschnittenen Knochenschaftes 132 eingeführt. Die Verbindung mit dem Knochen kann zementfrei oder mit geeigneten Befestigungsmitteln erfolgen.

Das Gelenk des Implantates weist eine kugelförmige Gleitfläche 111 auf, um die sich das zweite Gelenkteil 120 mit drei Freiheitsgraden bewegen kann. In anderen Worten lässt sich das zweite Gelenkteil 120 auf dem Gelenkkopf des ersten Gelenkteiles 110 frei bewegen. Eine Begrenzung der Bewegung findet erst statt, wenn der radial umlaufende Randbereich gegen den Marknagel 160 anläuft. Diese Beweglichkeit kann je nach Verlauf des Weichteilgewebes (nicht gezeigt in Figur 2) weiterhin eingeschränkt werden.

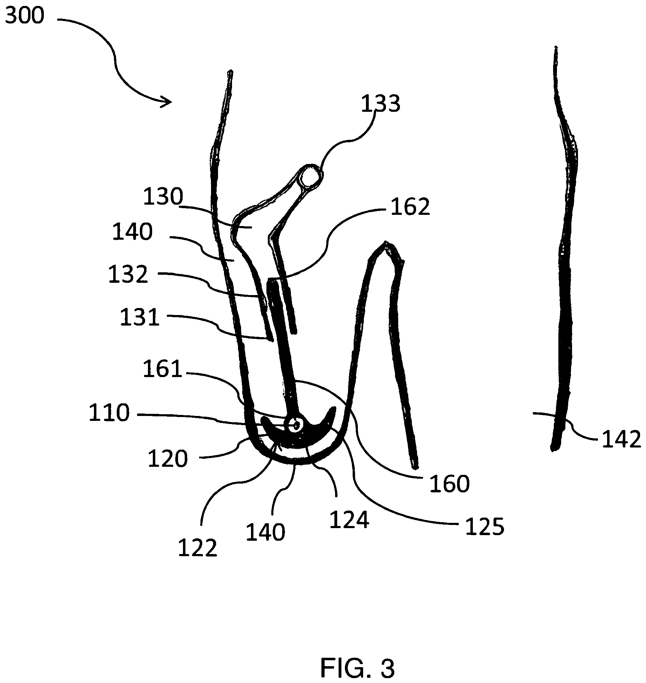

Figur 3 zeigt ein Femurprothesensystem 300 mit einem Marknagel 160, dessen proximales Marknagelende 162 in einen Oberschenkel 142 eines Patienten eingesetzt wurde. Das proximale Marknagelende 162 kann in einem natürlichen Femurknochen 130 eingeführt werden. Der Knochenschaft 132 kann auf seiner proximalen Seite 132 mit einer Hüftendoprothese (nicht gezeigt) verbunden werden. Der noch intakte Knochenschaft 132 kann eine natürliche Anbindung der Muskeln aufweisen, sodass Bewegungen beispielsweise der Hüfte weiterhin ausgeführt werden können.

Das distale Marknagelende 161 ist mit dem ersten Gelenkteil 110 fest verbunden. Noch vorhandene Muskeln zur Streckung und Beugung des Beines können mit dem zweiten Gelenkteil 120 an mehreren Punkten verbunden werden, um Kippbewegungen des Endteiles 124 erzeugen zu können.

Wird das im wesentlichen senkrecht zum Marknagel stehende Endteil 124 mit Gewicht belastet, nachdem eine nicht gezeigte Exoprothese angeschlossen wurde, kann das zweite Gelenkteil 120 so kippen, dass das Endteil eine im wesentlichen horizontale Lage einnimmt und somit die natürliche Schiefstellung des Femurknochenschaftes 132 ausgleicht. Eine starre Endplatte würde dagegen bei Belastung am tiefsten Punkt Druck aufbauen, so dass durch die Punktbelastung unerwünschte Druckstellen entstehen können. Ist das Endteil 124 im Wesentlichen horizontal ausgerichtet, können die Kräfte flächig vom Gelenkteil 120 aufgenommen werden. Da die Last auf der gesamten Außenfläche 122 verteilt wird, kann Druckstellen effektiv vorgebeugt werden.

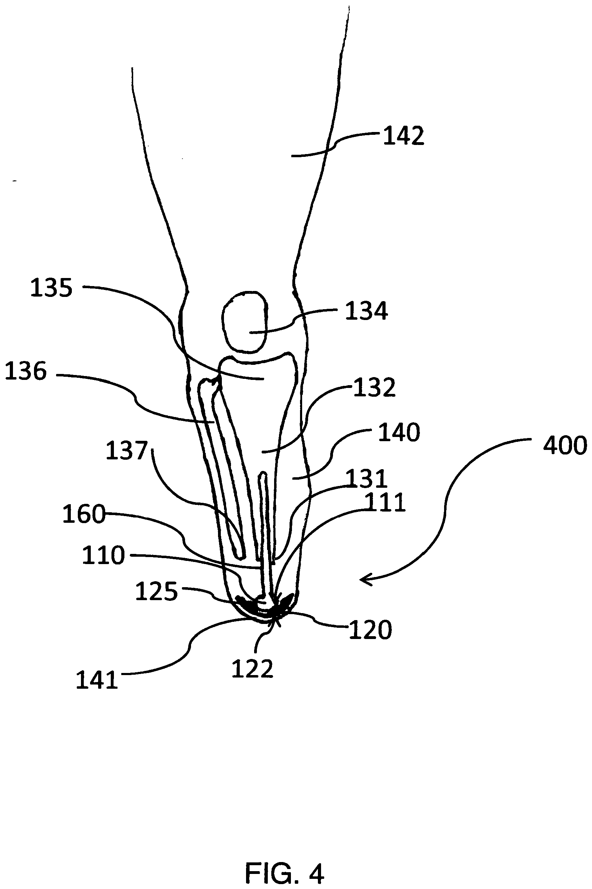

Figur 4 zeigt ein Prothesensystem 400 für einen Unterschenkel. Unterhalb des Oberschenkels 142 wird schematisch eine Kniescheibe 134 und daran anschließende Unterschenkelknochen, die Tibia 135 und die Fibula 136 gezeigt. Das distale Tibiaschaftende 131 ist mit einem Marknagel 160 verbunden. Der Marknagel kann hier in oder auf das distale Ende der Tibia angebracht werden, weil die Belastung vorrangig über den stärkeren Tibiaknochen übertragen wird. In der gezeigten Ausführungsform ist das distale Fibulaschaftende 137 nicht mit dem Prothesensystem direkt verbunden. Der radial umlaufende Randbereich 125 ist so ausgelegt, dass der Rand des zweiten Gelenkteiles 120 über die Fibula 136 hinausragt. In Abhängigkeit von der Länge des Fibulaknochens kann die Endplatte alternativ zum gezeigten Ausführungsbeispiel auch kleiner sein und das Ende der Fibula nicht überschreiten. Auch hier kann durch die Anbindung der Muskulatur sowohl eine Kippung als auch Drehung der Endplatte erfolgen.

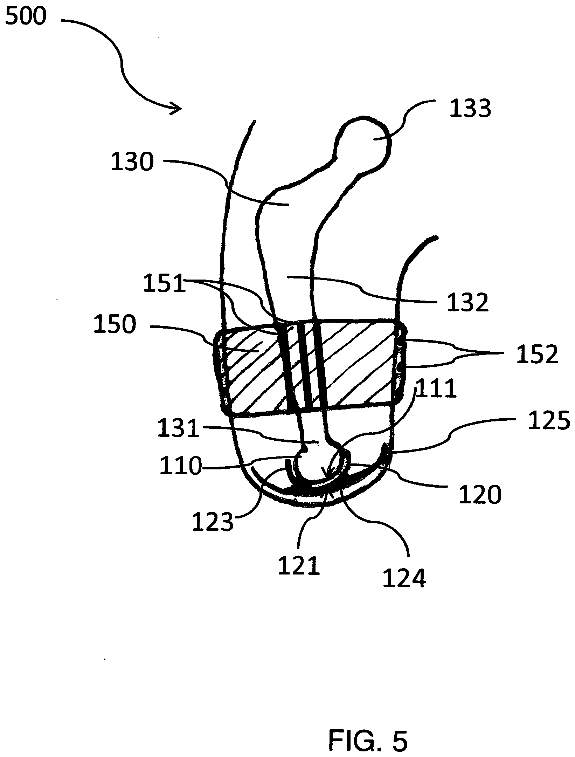

Figur 5 zeigt einen Oberschenkelstumpf mit einem Prothesensystem 500, wobei die Gleitfläche 121 einen ringförmigen Überstand 123 als Gelenkpfannenrand aufweist. Dieser Überstand 123 kann so ausgebildet sein, dass die Gelenkpfanne des zweiten Gelenkteiles 120 und die Gelenkkugel des ersten Gelenkteiles 110 eine lösbare Steckverbindung bildet. Hierzu ist der Überstand 123 vorzugsweise flexibel und/oder mit Federmitteln ausgebildet. Nach dem Einschnappen der Gelenkkugel in die Gelenkpfanne wird die Beweglichkeit des zweiten Gelenkteiles nicht eingeschränkt.

Ferner zeigt Figur 5 ein umlaufendes Band 150 zur Befestigung eines Schaftes (nicht gezeigt). Das Band kann aus hautverträglichen Materialien wie Stoff, Silikon oder andere geeignete Materialien ausgebildet sein. Bevorzugt werden Materialien verwendet, die auf der Haut haften, um den Halt zu verbessern. Das Band 150 kann wie eine Bandage festgezogen werden, um einen Schaft möglichst stabil mit dem Stumpf zu verbinden.

Das Band weist zudem als Haltemittel Haltevorsprünge 151 und Haltevertiefungen 152 auf, um mit der Rahmenkonstruktion eines Schaftes lösbar verbindbar zu sein. Als Haltemittel können übliche Formen verwendet werden, die mit einer entsprechenden Gegenform im Schaft in Eingriff gebracht werden können (z.B. Zähne, Rillen). Das Band und die Haltemittel dienen im Eingriff mit den entsprechenden Haltevorrichtungen am Schaft dazu, eine Rotationsstabilität des angezogenen Schaftes herzustellen.

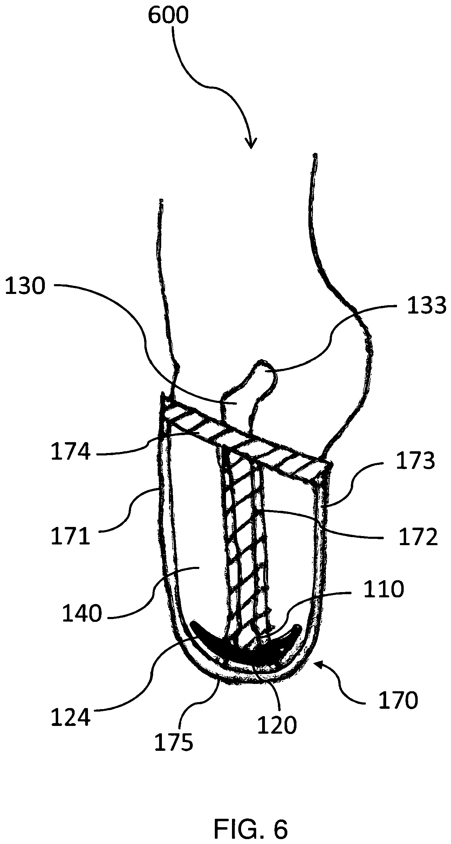

Figur 6 zeigt einen Rahmenschaft 170 für ein Femurprothesensystem 600. Das in Figur 5 gezeigte zirkuläre Band 150 wird zur besseren Darstellbarkeit des Schaft-Prothesensystems nicht gezeigt. Das Band 150 kann beispielsweise mit den in Figur 6 gezeigten Haltemitteln 152 am Arm 172 des Rahmenschaftes 170 befestigt werden. Weitere Arme 171 und 172 können zur stabilen Befestigung des Schaftes 170 an dem Band 150 (nicht gezeigt) dienen. Zudem wird zur proximalen Fixierung des Rahmenschaftes 170 ein umlaufender Schaftrand 174 bereitgestellt, welcher sowohl starr, flexibel oder auch teilflexibel ausgebildet sein kann.

Das offene Schaftkonzept mit Rahmenkonstruktion und einzelnen Armen 171, 172 und 173 ermöglicht einen Austausch von Luft und Temperatur mit der natürlichen Umgebung. Dies erhöht den Tragekomfort für den Nutzer im Vergleich zu Schaftsystemen, die zum großen Teil oder vollkommen geschlossen sind. Der offene Schaft schränkt den Nutzer im Gegensatz zu herkömmlichen geschlossenen Schaftsystemen in seiner Bewegung weder durch Material noch durch Zuschnitt ein.

Eine Gegenplatte 175, die an die Außenform des zweiten Gelenkteiles 120 angepasst ist, ist im unteren Bereich des Rahmenschaftes angeordnet. Die Gegenplatte 175 ermöglicht einen passgenauen Schaft. Dadurch kann ein optimaler Halt erzielt werden, wobei die Gefahren von Druck- und Wundstellen reduziert werden. Dies ist insbesondere für die Versorgung von Diabetes-Patienten von Bedeutung, die Amputationen benötigen. Ferner kann das Prothesensystem auch bei Knochenkrebspatienten angewendet werden, wenn ein Teil eines Knochens entfernt werden muss. Allgemein kann mittels der distalen Lastübertragung über die Gegenplatte 175 für Prothesennutzer Komfortverlust durch Beckenschiefstand oder Verschleiß von Kleidung durch einen erhöhten konstruktiven Aufwand vermieden werden.

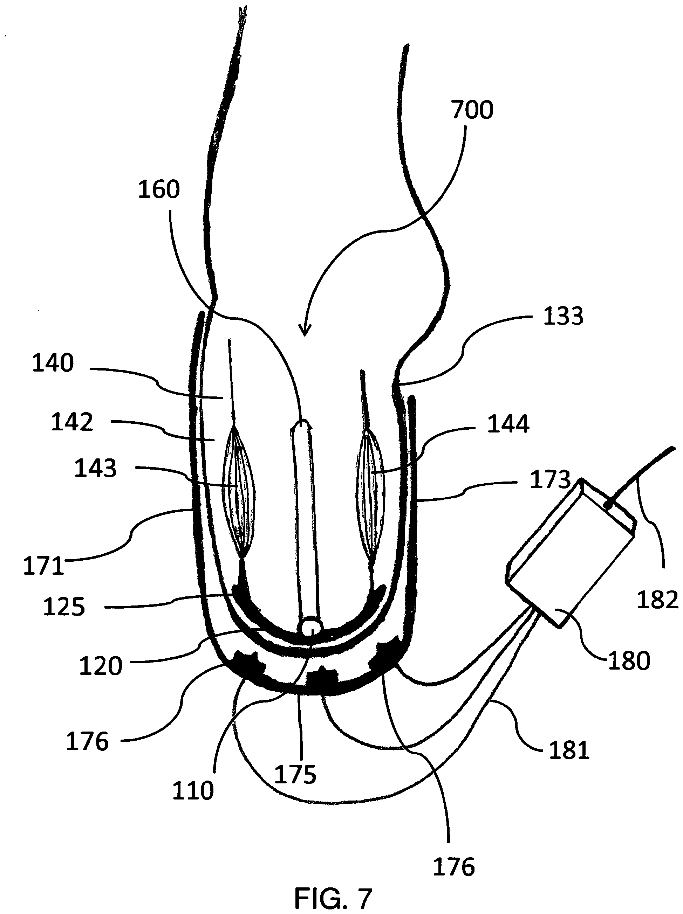

Figur 7 zeigt eine weitere Ausführungsform des Femurprothesensystems 700, wobei das System 700 in einer Schnittansicht von der Seite gezeigt wird. Der Schnitt ist durch einen Marknagel 160 gezeigt, der in einem Femurknochenschaft und/oder in einer Hüftendoprothese (nicht gezeigt) verankert ist.

In der gezeigten Schnittansicht werden für die Steuerung der Bewegungen einer extrakorporalen Prothese (nicht gezeigt) relevante Muskeln schematisch gezeichnet. Auf der Vorderseite des Oberschenkels 142 befinden sich streckende Muskeln 143, während auf der Rückseite des Unterschenkels beugende Muskeln 144 schematisch gezeigt sind. Diese Muskelgruppen 143 und 144 können mit Hilfe geeigneter Haltevorrichtungen oder mittels Löchern an den radialen Randbereich 125 des zweiten Gelenkteiles 120 befestigt werden. Die Muskeln 143, 144 können beispielsweise an die Unterseite des radialen Randbereiches 125 genäht werden. Hierzu können Löcher im radialen Randbereich 125 ausgebildet sein. Je nach Muskellage kann ein geeignetes Loch zur Anbindung ausgewählt werden, um einen Muskelansatz aufzunehmen. Alternativ oder zusätzlich zum Annähen kann die Muskulatur auch an hierzu geeigneten Flächen anwachsen bzw. mit geeignetem Material verwachsen.

Die Muskeln können willentlich von dem Prothesennutzer so angespannt werden, dass eine Kippung des zweiten Gelenkteiles 120 erfolgt. Diese Kippung kann durch die Messsensoren 176 erfasst werden. Kippt die Endplatte 124 beispielsweise durch Anspannung des hinteren Muskels 144 hinten nach oben und vorne nach unten, wird auf den vorderen Drucksensor 176 Druck ausgeübt. Auf diese Weise kann die Lage des zweiten Gelenkteiles 120 bestimmt werden. Die erfassten Messsignale werden über Verbindungsleitungen, die vorzugsweise im Schaft integriert werden, zu einer Steuereinheit 180 weitergeleitet. Auf diese Weise kann eine Beugung des Knies einer extrakorporalen Beinprothese gesteuert werden.

Die Steuereinheit 180 weist wenigstens einen Mikroprozessor auf und eine Sendeeinheit 182. Alternativ zu einer Sendeeinheit kann das Signal für die Steuerung über eine Kabelverbindung oder sonstige geeignete Mittel an die Steuereinheit übertragen werden. Die gezeigten Komponenten sind zu Veranschaulichung nicht maßstabsgetreu dargestellt und sind für die Anwendung so dimensioniert, dass sie in dem Schaft oder der Exoprothese integrierbar sind. Auf Grundlage der erfassten Messdaten können Steuersignale an Komponenten einer extrakorporalen Prothese gesendet werden. Die Übertragung der Messdaten kann statt über Signalleitungen 181 auch kabellos über übliche Sende- und Empfangsvorrichtungen erfolgen. Für die Bewegung der angesteuerten extrakorporalen Prothesenelemente können übliche Aktuatoren wie Ventile oder Motoren verwendet werden.

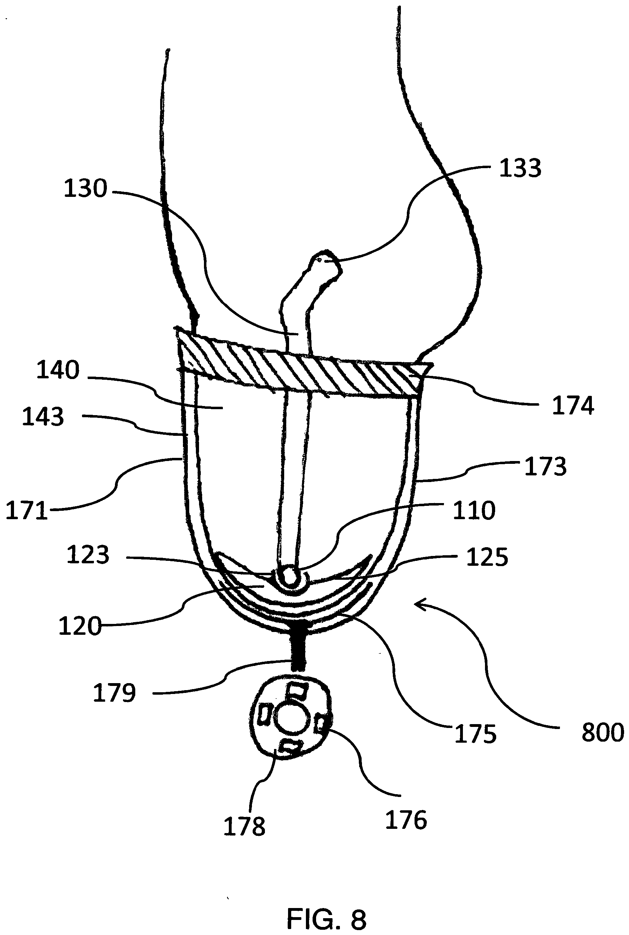

Figur 8 zeigt eine seitliche Schnittansicht einer weiteren Ausführungsform eines Prothesensystems 800 mit Rahmenschaft 170 zusammen mit einer Draufsicht eines externen Messsystems, das unterhalb des Schaftes angebracht ist. Das Messsystem ist in Form eines Rohrelementes 178 ausgeführt ist, wobei das Rohrelement 178 einen Stift 179, der mit der Gegenplatte 175 verbunden ist, aufnehmen kann. Die Bewegung der im Schaft befindlichen Gegenplatte 175 zwischen Stumpf und Rahmen kann mittels geeigneter Sensoren 176 gemessen werden. Wie in der schematischen Draufsicht in Figur 8 dargestellt, kann das Rohrelement 178 eine Mehrzahl von Messsensoren 176 aufweisen.

Dieses Messsystem stellt eine alternative oder weitere Messmöglichkeit für die Bewegungen der Endplatte 124 dar. Dabei wird die Bewegung der Endplatte 124 auf die als Zwischenplatte fungierende Gegenplatte 175 übertragen. Das externe Messsystem kann auf diese Weise die Bewegung eines beweglichen Anteils im Schaft - hier die Gegenplatte 175 - und/oder die Belastung der Prothese messen.

Figur 8 zeigt eine Femurprothesensystem, wobei die Lage des um den oberen Oberschenkel verlaufenden Schaftrand 174 schematisch angezeigt wird. Im Weichteilmantel 140 des Oberschenkels befindet sich der Femurknochen 130 mit Femurkopf 133.

Der erste Gelenkteil 110 liegt als Gelenkkopf in der Gelenkpfanne des zweiten Gelenkteiles 120. Die Gelenkpfanne wird durch den ringförmigen Überstand 123 begrenzt. Weiterhin weist das zweite Gelenkteil 120 einen radial umlaufenden Randbereich 125 auf. Die Gegenplatte 175 ist an die Außenform des subkutanen zweiten Gelenkteiles 120 angepasst.

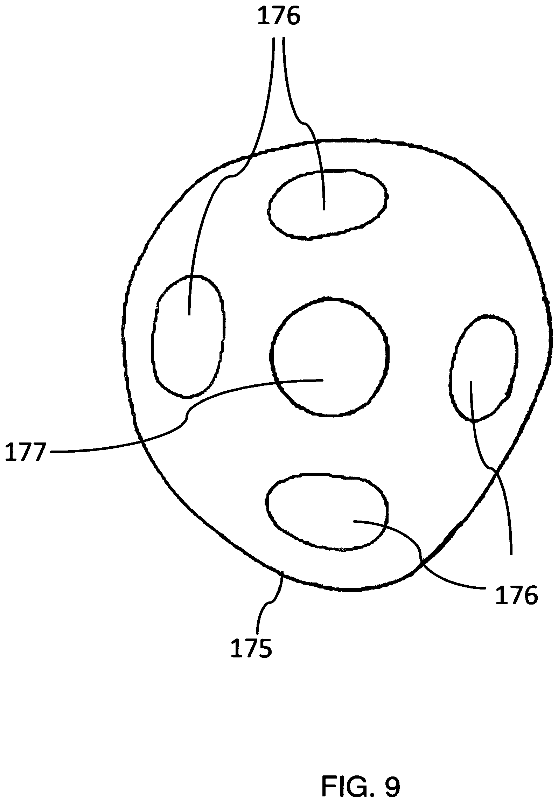

Figur 9 zeigt eine Detailansicht eines externen Messsystems in Form einer Gegenplatte 175 (vgl. Figur 7). Im radialen Randbereich der Gegenplatte 175 sind Drucksensoren angeordnet, um die Lage bzw. Lageveränderungen des zweiten Gelenkteiles 120 erfassen zu können. Im Zentrum 177 können axiale Kräfte gemessen werden. Über das Zentrum 177 der Gegenplatte kann gegebenfalls eine Exoprothese verbunden werden (vgl. Stift 179 in Figur 8), sowie gegebenfalls Signalleitungen zu steuerbaren Komponenten geführt werden.

Die Erfindung ist nicht beschränkt auf die gezeigten Ausführungsformen, sondern kann auch für andere amputierte oder exartikulierte Gliedmaßen verwendet werden.

### Bezugszeichenliste

- 100: Prothesensystem
- 101: Implantat
- 110: Erstes Gelenkteil
- 111: Gleitfläche des ersten Gelenkteiles
- 120: Zweites Gelenkteil
- 121: Gleitfläche der zweiten Gelenkteils
- 122: Außenfläche des Endteiles
- 123: Ringförmiger Überstand
- 124: Endteil des zweiten Gelenkteiles
- 125: Radial umlaufender Randbereich
- 130: Femurknochen
- 131: Distales Schaftende
- 132: Knochenschaft
- 133: Femurkopf
- 134: Kniescheibe
- 135: Tibia
- 136: Fibula
- 137: Distales Fibulaschaftende
- 140: Weichteilmantel
- 141: Distales Amputationsstumpfende
- 142: Oberschenkel
- 143: Streckende Muskeln
- 144: Beugende Muskeln
- 150: Umlaufende Band
- 151: Haltevorsprünge
- 152: Haltevertiefungen
- 160: Marknagel
- 161: Distales Marknagelende
- 162: Proximales Marknagelende
- 170: Rahmenschaft
- 171: Arm des Rahmenschaftes
- 172: Weiterer Arm des Rahmenschaftes
- 173: Weiterer Arm
- 174: Umlaufender Schaftrand
- 175: Gegenplatte
- 176: Messsensoren
- 177: Zentrum der Gegenplatte
- 178: Rohrelement
- 179: Stift
- 180: Steuereinheit
- 181: Signalleitung
- 182: Sendeeinheit
- 200: Prothesensystem mit Marknagel
- 300: Femurprothesensystems
- 400: Tibiaprothesensystem
- 500: Band für ein Femurprothesensystem
- 600: Prothesensystem mit Schaft
- 700: Weiteres Prothesensystem mit Schaft
- 800: Weiteres Prothesensystem mit Schaft

## Patentansprüche

1. Prothesensystem für menschliche Gliedmaße aufweisend
ein Implantat (101) mit mindestens zwei zueinander bewegbaren Gelenkteilen, wobei das erste Gelenkteil (110) mit einem Knochenteil (131, 132) verbindbar ist, **dadurch gekennzeichnet, dass**
das zweite Gelenkteil (120) auf dessen gelenkfernen Seite ein subkutan anzuordnendes Endteil (124) aufweist.

2. Prothesensystem nach Anspruch 1,
wobei das Endteil (124) plattenförmig ausgebildet ist.

3. Prothesensystem nach einem der Ansprüche 1 und 2, wobei das Endteil eine konvex gekrümmte Außenfläche (122) aufweist.

4. Prothesensystem nach einem der vorhergehenden Ansprüche,
wobei das erste Gelenkteil (110) in Form eines Gelenkkopfes und das zweite Gelenkteil (120) in Form einer Gelenkpfanne ausgebildet ist.

5. Prothesensystem nach einem der vorhergehenden Ansprüche,
wobei das erste Gelenkteil (110) hohlförmig ausgebildet ist und Befestigungsmittel zur Verbindung mit dem Knochenteil (131) aufweist, die ausgewählt sind aus der Gruppe umfassend knochenaufbauendes Material, Knochenzement und Haftmittel.

6. Prothesensystem nach einem der Ansprüche 1 bis 4, wobei das erste Gelenkteil (110) über einen Marknagel (160) und/oder eine Verschraubeinheit mit einem Knochenteil (132) verbindbar ist.

7. Prothesensystem nach einem der vorhergehenden Ansprüche, wobei das Endteil (124) einen radial umlaufenden Randbereich (125) aufweist.

8. Prothesensystem nach einem der vorhergehenden Ansprüche,
wobei das Endteil (124) des zweiten Gelenkteiles (120) Flächen und/oder Material zur Anbindung von Muskeln aufweist.

9. Prothesensystem nach einem der vorhergehenden Ansprüche, ferner aufweisend einen den Gliedmaßenstumpf des Prothesennutzers wenigstens teilweise umhüllenden Schaft (170) zur Anbindung an extrakorporale Prothesenmechanismen.

10. Prothesensystem nach Anspruch 9, wobei der Schaft (170) eine Gegenplatte (175) aufweist, deren Form an die Außenform des zweiten Gelenkteiles (120) angepasst ist.

11. Prothesensystem nach Anspruch 9 oder 10, wobei der Schaft (170) als Rahmenschaft ausgebildet ist.

12. Prothesensystem nach einem der Ansprüche 9 bis 11, wobei der Schaft (170) ein zirkulär verlaufendes Band (150) als Haltevorrichtung für den Schaft aufweist.

13. Prothesensystem nach einem der Ansprüche 1 bis 12, wobei wenigstens ein externes Messsystem (178) bereitgestellt wird, das Messsensoren (176) zum Erfassen der Position des zweiten Gelenkteiles (120) aufweist.

14. Prothesensystem nach Anspruch 13, wobei das wenigstens eine externes Messsystem als Gegenplatte (175) und/oder Rohrelement (178) ausgebildet ist.

15. Prothesensystem nach einem der vorhergehenden Ansprüche, wobei wenigstens ein internes Messsystem bereitgestellt wird, um die Position und/ oder Belastung wenigstens des zweiten Gelenkteiles (120) zu erfassen.

16. Prothesensystem nach einem der Ansprüche 13 bis 15, ferner aufweisend eine Steuereinheit (180), wobei auf Grundlage der erfassten Messsignale extrakorporale Prothesenmechanismen steuerbar sind.

## Claims

1. A prosthesis system for human limbs having
an implant (101) having at least two mutually movable joint members, wherein the first joint member (110) can be connected to a bone portion (131, 132),
**characterised in that**
the second joint member (120) has an end portion (124), which is intended to be arranged subcutaneously, on the side thereof remote from the joint.

2. The prosthesis system according to claim 1, wherein the end portion (124) is configured in a plate-shaped manner.

3. The prosthesis system according to any one of claims 1 and 2, wherein the end portion has a convex-curved outer face (122).

4. The prosthesis system according to any one of the preceding claims, wherein the first joint member (110) is configured in the form of an articular head and the second joint member (120) is configured in the form of an articular socket.

5. The prosthesis system according to any one of the preceding claims, wherein the first joint member (110) is configured in a hollow manner and has fixing means for connection to the bone portion (131) which are selected from the group consisting of bone-forming material, bone cement and adhesive.

6. The prosthesis system according to any one of claims 1 to 4, wherein the first joint member (110) can be connected to a bone portion (132) via a medullary pin (160) and/or a screw unit.

7. The prosthesis system according to any one of the preceding claims, wherein the end portion (124) has a radially circumferential edge region (125).

8. The prosthesis system according to any one of the preceding claims, wherein the end portion (124) of the second joint member (120) has faces and/or material for connecting muscles.

9. The prosthesis system according to any one of the preceding claims, further having a shaft (170) which at least partially surrounds the limb stump of the prosthesis user in order to be connected to extracorporeal prosthesis mechanisms.

10. The prosthesis system according to claim 9, wherein the shaft (170) has a counter-plate (175), the form of which is adapted to the outer form of the second joint member (120).

11. The prosthesis system according to claim 9 or 10, wherein the shaft (170) is configured as a frame shaft.

12. The prosthesis system according to any one of claims 9 to 11, wherein the shaft (170) has a band (150) extending in a circular manner as a retention device for the shaft.

13. The prosthesis system according to any one of claims 1 to 12, wherein there is provided at least one external measurement system (178) which has measurement sensors (176) for detecting the position of the second joint member (120).

14. The prosthesis system according to claim 13, wherein the at least one external measurement system is configured as a counter-plate (175) and/or pipe element (178).

15. The prosthesis system according to any one of the preceding claims, wherein at least one internal measurement system is provided in order to detect the position and/or loading of at least the second joint member (120).

16. The prosthesis system according to any one of claims 13 to 15, further having a control unit (180), wherein extracorporeal prosthesis mechanisms can be controlled on the basis of the measurement signals detected.

## Revendications

1. Système de prothèse pour membres humains comprenant
un implant (101) avec au moins deux parties articulées mobiles l'une par rapport à l'autre,
dans lequel la première partie articulée (110) peut être reliée à une partie formant os (131, 132),
**caractérisé en ce que**
la deuxième partie articulée (120) comprend sur sa face opposée à l'articulation une partie terminale (124) destinée à être disposée sous la peau.

2. Système de prothèse selon la revendication 1, dans lequel la partie terminale (124) est conçue en forme de plaque.

3. Système de prothèse selon l'une des revendications 1 et 2, dans lequel la partie terminale comprend une surface externe (122) courbée de manière convexe.

4. Système de prothèse selon l'une des revendications précédentes, dans lequel la première partie articulée (110) est conçue sous la forme d'une tête articulée et la deuxième partie articulée (120) est conçue sous la forme d'une coque acétabulaire.

5. Système de prothèse selon l'une des revendications précédentes, dans lequel la première partie articulée (110) est de conception creuse et comprend des moyens de fixation pour la liaison à la partie formant os (131), qui sont sélectionnés dans le groupe comportant un matériau impliqué dans la formation osseuse, un ciment osseux et un adhésif.

6. Système de prothèse selon l'une des revendications 1 à 4, dans lequel la première partie articulée (110) peut être reliée à une partie formant os (132) par l'intermédiaire d'un clou intramédullaire (160) et/ou d'une unité de vissage.

7. Système de prothèse selon l'une des revendications précédentes, dans lequel la partie terminale (124) comprend une zone de bordure radialement périphérique (125).

8. Système de prothèse selon l'une des revendications précédentes, dans lequel la partie terminale (124) de la deuxième partie articulée (120) comprend des surfaces et/ou un matériau pour le rattachement de muscles.

9. Système de prothèse selon l'une des revendications précédentes, comprenant en outre une tige (170) enveloppant au moins en partie le moignon de membre de l'utilisateur de la prothèse pour le rattachement à des mécanismes de prothèse extracorporels.

10. Système de prothèse selon la revendication 9, dans lequel la tige (170) comprend une contre-plaque (175) dont la forme est adaptée à la forme externe de la deuxième partie articulée (120).

11. Système de prothèse selon la revendication 9 ou la revendication 10, dans lequel la tige (170) est conçue sous la forme d'une tige de cadre.

12. Système de prothèse selon l'une des revendications 9 à 11, dans lequel la tige (170) comprend une bande s'étendant circulairement (150) en tant que dispositif de support pour la tige.

13. Système de prothèse selon l'une des revendications 1 à 12, dans lequel est fourni au moins un système de mesure externe (178) qui comprend des capteurs de mesure (176) pour la détection de la position de la deuxième partie articulée (120).

14. Système de prothèse selon la revendication 13, dans lequel l'au moins un système de mesure externe est conçu sous la forme d'une contre-plaque (175) et/ou d'un élément tubulaire (178).

15. Système de prothèse selon l'une des revendications précédentes, dans lequel est fourni au moins un système de mesure interne pour détecter la position et/ou la charge d'au moins la deuxième partie articulée (120).

16. Système de prothèse selon l'une des revendications 13 à 15, comprenant en outre une unité de commande (180), dans lequel des mécanismes de prothèse extracorporels peuvent être commandés sur la base des signaux de mesure détectés.
